# EUROPÄISCHE PATENTSCHRIFT

(11) **EP 3 585 194 B1**
(45) Veröffentlichungstag und Bekanntmachung des Hinweises auf die Patenterteilung: **13.04.2022**
(21) Anmeldenummer: 18706452.2
(22) Anmeldetag: 13.02.2018
(51) Int. Cl.: A01K 1/00, A41D 13/005, A62B 17/00, A61F 7/00, A61F 7/02, A01K 13/00

(54) **TRAGBARE KÜHLVORRICHTUNG FÜR MENSCHEN UND/ODER TIERE**
PORTABLE COOLING DEVICE FOR HUMANS AND/OR ANIMALS
DISPOSITIF DE REFROIDISSEMENT PORTATIF POUR HUMAINS ET/OU ANIMAUX

(30) Priorität: 23.02.2017 DE 102017103731
(43) Veröffentlichungstag der Anmeldung: 01.01.2020
(73) Patentinhaber: Labus, Heinrich Raimund, 40789 Monheim (DE)
(72) Erfinder: LABUS, Andreas, 51399 Burscheid (DE); LABUS, Heinrich Raimund, 40789 Monheim (DE)
(74) Vertreter: Schoenen, Norbert
(86) Internationale Anmeldenummer: PCT/EP2018/053517
(87) Internationale Veröffentlichungsnummer: WO 2018/153724

(56) Entgegenhaltungen:
- EP-A1- 0 490 347
- JP-A- 2001 040 512
- US-A- 5 217 408
- US-A1- 2002 170 309
- US-A1- 2013 319 031

## Beschreibung

Die Erfindung betrifft eine tragbare Kühlvorrichtung für Menschen und/oder Tiere zum Anbringen an den Oberkörper- und Kopfbereich, in Gestalt eines Umhanges, einer Jacke bzw. Weste oder Decke zum Schutz bei hoher Umgebungstemperatur.

Die Erfindung betrifft insbesondere eine Kühlvorrichtung für den Oberkörper eines Menschen, und entsprechend modifiziert auch für die Vorderkörper von Haustieren, insbesondere für Hunde und Pferde, zum Schutz bei hoher Umgebungstemperatur.

Bei Aktivitäten von Mensch und Tier in übermäßig warmer und in der Regel auch sonniger Umgebung wehrt sich der Körper vor Überhitzung durch Schwitzen, um die erhöhte Körpertemperatur wieder auf ein normales Niveau zu senken und zu stabilisieren. Über einen längeren Zeitraum kann ein solcher körperbelastender Zustand allerdings gesundheitsschädlich oder sogar lebensbedrohend sein, weil neben gefährlicher Überhitzung dabei auch relativ viel Flüssigkeit und lebensnotwendige Mineralien nebst anderer Substanzen permanent ausgeschieden werden. Von den infrage kommenden Haustieren sind insbesondere Hunde gegen Hitze anfällig, weil sie die überschüssige Wärme nur geringfügig über Schwitzen abgeben können. Der überwiegende Teil der Wärmeabgabe findet bei Hunden hauptsächlich durch Hecheln statt, was wegen der geringen Verdunstungsfläche nicht besonders effektiv ist.

### Stand der Technik

Es sind aus der Patentliteratur einige Lösungsprinzipien für den individuellen Schutz des menschlichen bzw. tierischen Körpers vor Überhitzung bekannt. Die dort beschriebenen Vorrichtungen für Menschen werden meistens in Form einer Weste konzipiert. Eine der ersten Kühlvorrichtungen basiert auf kontinuierlicher Belüftung des Raumes zwischen dem Oberkörper und einer Jacke mit eingebauten Ventilatoren (EP 490 347 A1). Ein anderes Kühlkonzept beruht auf einer Weste mit porösen Einlagen und Ventilatoren. Die Einlagen dienen hier als Abstandshalter und zugleich als Luftpassage für die Belüftung des Oberkörpers. Bei Bedarf kann alternativ kalte oder erwärmte Luft eingesetzt werden (US 2007/0118956).

Bekannt ist auch eine Vorrichtung zur Klimatisierung des menschlichen Körpers in Gestalt eines Mantels mit daran angebundener Kopfbedeckung, ausgestattet mit einer komplexen Inneninstallation. Die Belüftung erfolgt mit Außenluft mit Hilfe von: Ventilator, Heizelement und Batterie, welche in einer externen Box untergebracht sind. Das Heizelement kann nach Bedarf für die Erwärmung der eingesetzten Luft zugeschaltet werden (US 3,468,299 1969).

Ein anderes Prinzip zur Kühlung des Oberkörpers beruht auf im Kreislauf gepumpter Luft in dafür vorgesehenen Kanälen in der Weste. Die darin zirkulierende Luft entzieht im Kontakt mit dem Oberkörper stetig einen Teil der überschüssigen Wärme sowie Schweißfeuchte und strömt anschließend in eine Klimabox, wo sie nach Trocknung und Abkühlung wieder in den Umlauf geführt wird (WO 2014/031 128 A1).

Ein anderes Patent schützt eine klimatisierte Sicherheitsweste mit zusätzlicher Schutzausstattung für Sondereinsätze. Die Klimatisierung des Benutzers geschieht durch Belüftung mit Hilfe von mehreren Lüftern mit Außenluft, welche nach Bedarf elektrisch erwärmt werden kann. Die Elektrobatterie wird nachgeladen durch die, im hinteren Westenbereich angebrachte, Solarpanel (US 2012/0036623 A1).

Ein grundsätzlich anderes Kühlprinzip, und zwar ausschließlich nur mit Wasser, unterscheidet sich wesentlich von den oben beschriebenen Funktionsweisen. Demnach besteht die Kühlvorrichtung zwar auch in Gestalt einer Weste, allerdings aus einem Gewebe mit Zusätzen, die reversibel wasserspeichernde Eigenschaften aufweisen. Das zuvor in die Weste periodisch vor dem Kühleinsatz eingebrachte Wasser verdunstet dann allmählich während der Kühlung und entzieht dabei die überschüssige Wärme aus dem zu kühlenden Bereich (DE 10 2011 016 482 A1). Die nach diesem Prinzip funktionierenden Kühlwesten werden kommerziell für bestimmte berufliche Tätigkeiten bzw. für Motorradfahrer oder für Tiere, wie Hunde oder Pferde in Gestalt von Kühlmantel bzw. Kühldecke, angeboten.

Nach dem Kühlprinzip der Oberkörperbelüftung arbeitet auch die Vorrichtung, beschrieben im Patent US 2013/0319031 A1. Für effektive Luftverteilung sind zwei übereinander angeordnete flache Kammern vorgesehen. Die Kühlvorrichtung ist hauptsächlich bestimmt für die Nutzung zusammen mit einer schusssicheren Weste. Die Belüftung erfolgt mit Hilfe von Lüfter und Batterie.

Eine andere tragbare Vorrichtung für Mensch, konzipiert zur Kühlung des Rückens, basiert auf Anwendung von Luft und zerstäubtem Wasser als Kühlmittel (US 6,543,247). Sie setzt sich zusammen aus einem elektrisch angetriebenen Lüfter und einem Wasserzerstäuber mit konventioneller Luft/Wasser-Düse. Diese Bauteile sind untergebracht in einem Gehäuse mit verbreitetem Ausgang von Venturi-Typ für das ausströmende Kühlmedium. Das Wasser wird direkt in der Luftströmung des Lüfters zerstäubt, unter die Kleidung am Rücken entlang geleitet und kontinuierlich gekühlt. Das obige Kühlprinzip wurde auch schon früher in einer anderen Kühlvorrichtung als Handgerät realisiert und unter dem Handelsnahmen "Arctic Blast" vertrieben.

Bei den Kühlwesten mit erzwungener Luftzirkulation im Bereich zwischen dem Oberkörper und der zugewandten Seite der Kühlweste ist die Wirkung nicht nur von der Außentemperatur und der relativen Luftfeuchte, sondern auch von den körperlichen Merkmalen des konkreten Anwenders abhängig. Die abzuführende überschüssige Wärmemenge variiert nämlich von Mensch zu Mensch unterschiedlich stark, je nach Körpergewicht, Größe, Alter, physiologischen Besonderheiten und hängt natürlich auch vom Gesundheitszustand und der aktuell stattfindenden Aktivität der zu kühlenden Person ab. Neben der Luftgeschwindigkeit am Oberkörper während des Kühlvorgangs übt die sich bildende Schweißmenge einen wesentlichen Einfluss auf die Kühleffektivität aus, insbesondere bei Temperaturen der verwendeten Außenluft oberhalb von etwa 35 °C.

Bei den bekannten Vorrichtungen nach dem Prinzip der Kühlung mit Umgebungsluft besteht leider keine Möglichkeit, diese Faktoren zu kompensieren.

Auch die Kühlwesten nach dem Prinzip der Wasserspeicherung im Gewebe mit anschließender Verdunstung sind mit vielen Nachteilen behaftet. Da die Wasserverdunstung und somit auch die dadurch bewirkte Senkung der Temperatur direkt auf der Oberfläche des nassen Gewebes geschehen, findet die Abkühlung des nassen Westengewebes bevorzugt statt. Die angestrebte Kühlung des menschlichen Körpers läuft deswegen indirekt und zeitverzögert ab. Aufgrund der hier fehlenden Belüftung und dem daraus resultierenden allmählichen Anstieg der Feuchte im Raum zwischen dem Kühlgewebe und dem zu kühlenden Körper ist der Kühlvorgang zusätzlich beeinträchtigt. Darüber hinaus kann der sich bildende Schweiß nur erschwert oder kaum verdunsten und entweichen. Hinzu kommt, dass die Kühlung nicht steuerbar sei.

Nachteilig ist auch das hohe Anfangsgewicht der Weste aufgrund der relativ großen gespeicherten Wassermenge und des recht hohen und ineffektiven Wasserverbrauchs. Die Anwendung derartiger Kühltechnik in trockener Gegend ist wegen des dort in der Regel herrschenden Wassermangels naturgemäß eingeschränkt. Einer der gravierenden Nachteile der bekannten Kühlvorrichtungen mit Luft/Wassergemisch als Kühlmedium sind die verhältnismäßig großen Wassertropfen, die für die dort vorgesehen konventionellen Luft/Wasserdüsen spezifisch sind. Die Größe der Tropfen streut außerdem im breiten Bereich von etwa 100 bis 20 µm (Quelle: Ecologic Technologies, Inc., 2017). Hinzu kommt, dass das Einleiten des zerstäubten Wassers in die Luftströmung den Zusammenschluss kleinerer Tropfen zu größeren Tropfen befördert. Nach Erreichen einer kritischen Größe und somit Masse, scheiden diese Tropfen aus dem Luftstrom deshalb aus und werden am weiteren Kühlvorgang nur unwesentlich oder gar nicht mehr beteiligt. Auch die meisten von den übrigen Tropfen werden dabei im Durchschnitt grösser, deren Gesamtoberfläche im Vergleich zum Anfangszustand dadurch wesentlich kleiner und die Effektivität der Kühlung dementsprechend schlechter. Die ausgeschiedenen Wassertropfen sammeln sich in der Vorrichtung und können öfter zu hygienischen Problemen führen. Ein weiterer Nachteil ist die nicht regelbare Luftmenge, weshalb die Kühleffizienz sich nicht optimal anpassen lässt. Bei überdurchschnittlich hoher Außentemperatur besteht nämlich das Problem, das die bei übermäßig starkem Luftzustrom enthaltene Wärme die Kühlwirkung des zerstäubten Wassers nivellieren oder sogar zur zusätzlichen Erwärmung des Oberkörpers beitragen würde. Infolge der unbefriedigenden Kühlwirkung und zum Teil auch wegen der Komplexität der aus dem Stand der Technik bekannten Vorrichtungen sind diese insbesondere für Aktivitäten im Sport- und Freizeitbereich kaum geeignet.

### Aufgabe und Lösung der Erfindung

Die Aufgabe der vorliegenden Erfindung liegt in der Bereitstellung von tragbaren Kühlvorrichtungen für Mensch und Tiere, insbesondere für Hunde und Pferde, konzipiert vor allem für eine überdurchschnittlich warme Umgebung. Die Kühlvorrichtungen sollen kompakt sowie einfach in Aufbau und Anwendung sein. Im sonnigen Gelände sollen sie weitgehend energetisch autark einsetzbar sein. Sie sollen die bestehende Lücke an geeigneten Kühlvorrichtungen für berufliche und andere Aktivitäten von Mensch und Tier im heißen Gelände füllen.

Diese Aufgabe wird durch eine tragbare Kühlvorrichtung nach Anspruch 1 gelöst. Die Ansprüche 2 bis 12 betreffen bevorzugte Ausführungsformen der Erfindung.

Unter anderem werden die folgenden Vorteile erreicht:
Die Kühlvorrichtungen für Menschen bzw. Tiere nach der Erfindung beruhen auf dem kontinuierlichen Entzug der überschüssigen Körperwärme durch kontinuierliches Zerstäuben und Verdampfen von Wasser sowie Austragung des Wasserdampfes durch einstellbare Belüftung des zu kühlenden Körperbereichs. Besonders wirksam ist die Kühlung der intensiv Wärme abgebenden Bereiche, wie die des Oberkörpers bei Menschen und die des Vorderkörpers bei Tieren. Die Kühlwirkung nach der Erfindung wird hauptsächlich durch Verdampfen des kontinuierlich mit Hilfe von Ultraschallwellen sehr fein zerstäubten Wassers erreicht und zwar in unmittelbarer Nähe des zu kühlenden Körperbereiches und des Kopfes. Durch die Kühlung infolge der Verdampfung des fein zerstäubten Wassers direkt in dem zu kühlenden Bereich und aufgrund der recht spärlichen Belüftung dieses Bereiches sind die Kühlvorrichtungen für Mensch und Tiere nach der Erfindung auch bei Außentemperaturen oberhalb von 35 °C in der Regel noch brauchbar. Die recht hohe Temperatur und Feuchte im zu kühlenden Bereich begünstigen natürlich eine Ansiedlung und Wachstum von Mikroorganismen. Um deren unerwünschten Ausbreitung vorzubeugen, wird nach der Erfindung, für die Zerstäuber ein salzfreies Wasser mit geringem Zusatz an Wasserstoffperoxid als Biozid verwendet, welches für Mensch und Tier bei diesen Umständen bedenkenlos sei.

In den Kühlvorrichtungen nach der Erfindung wird die Umgebungsluft zur Belüftung des Kühlbereiches und zum Austragen des gebildeten Wasserdampfes und nur untergeordnet als Kühlmittel benötigt. Ab einer Außentemperatur von etwa 35 °C muss daher der Luftfluss lediglich für eine ausreichende Belüftung des zu kühlenden Bereiches sorgen und kann darauf beschränkt bleiben. Die Kühlwirkung nach der Erfindung ist im Allgemeinen viel effektiver als nur mit Luft allein und ist auch bei Außentemperaturen oberhalb von 37 °C noch möglich. Infolge der besseren Kühlwirkung der Vorrichtung nach der Erfindung wird das lästige, übermäßige Schwitzen weitgehend unterdrückt.

Die für die Kühlung verwendete Umgebungsluft und das zerstäubte Wasser sind vorzugsweise mengenmäßig regelbar, um die Kühlleistung der aktuell herrschenden Außentemperatur anzupassen und optimal zu gestalten. In den Kühlvorrichtungen nach der Erfindung kann die Mengenregelung auf einfache Weise, durch Veränderung der elektrischen Spannung an den Lüftern und/oder den Wasserzerstäubern mit Hilfe von Spannungsreglern und zwar im kontinuierlichen Betrieb oder in wählbaren Zeitintervallen, vorgenommen werden.

Für die benötigte Elektroenergie zur Belüftung und zum Zerstäuben von Wasser ist die Vorrichtung nach der Erfindung mit einem Akkumulator ausgestattet. Der kann nach Bedarf aus dem Elektronetz periodisch aufgeladen und während des Betriebes in sonniger Gegend von einem Solarpanel kontinuierlich nachgeladen werden. Auf diese Weise können die Kühlvorrichtungen nach der Erfindung sogar stromautark betrieben werden. Zur Sicherung einer optimalen Stromausbeute wird das Solarpanel horizontal bzw. entsprechend dem Sonnenstand über dem Kopf ausgerichtet. Es können nach Bedarf auch zusätzlich oder alternativ kleinere Solarpanel an den Schultern positioniert werden.

Zum Schutz vor Außenwärme nach der Erfindung dient eine Umhüllung für den Oberkörper und einem Teil der Vorrichtung und zwar in Gestalt z. B. eines Umhangs oder einer Jacke. Das Hauptmerkmal dieser thermischen Abschirmung nach der Erfindung ist deren metallisierte oder eine andersartige, wärmereflektierende Außenoberfläche und eine thermische Isolierschicht auf der Unterseite der Umhüllung, angefertigt aus Gewebe und/oder Folie, und ein darüber liegendes, teils lichtdurchlässiges, mattes und dadurch lichtstreuendes, zusätzliches Gewebe und/oder eine entsprechende Folie. Die Abschirmung kann sonst beliebig gestaltet und dem Modetrend bzw. den örtlichen Kleidungsgewohnheiten angepasst und nach Bedarf gewechselt werden. Das nach außen gerichtete Gewebe bzw. die entsprechende Folie hat hauptsächlich eine maskierende Funktion, weil es die metallisierte Schicht verhüllt und dadurch praktisch keine störende Lichtreflexion wahrnehmbar wird. Der Zweck der metallbeschichteten Oberfläche der Abschirmung nach der Erfindung liegt darin, möglichst viel von der ankommenden Wärmestrahlung zu reflektieren und dadurch die Effektivität der Kühlvorrichtung zu verbessern.

Die Kühlvorrichtung für den Menschen nach der Erfindung basiert auf der Kühlung des Oberkörpers und des Kopfes, weil die überschüssige Wärme besonders intensiv in diesen Bereichen abgegeben wird. Sie besteht aus einem rucksackähnlichen Behältnis mit Tragegurten und einem daran angebrachten, zusammenlegbaren Gerüst als Träger für das Solarpanel und für eine im Kopfbereich positionierte Sonnenschutzblende mit darauf befestigtem Lüfter und Wasserzerstäuber.

Die dem Rücken zugewandte Wand des Behältnisses weist eine flies- bzw. filzähnliche, ausreichend grobstrukturierte Oberfläche auf, um in dem zu kühlenden Bereich zwischen dem Rücken und dieser Behältniswand optimale Bedingungen für die Verdampfung und somit eine möglichst hohe Effizienz der Kühlwirkung des zerstäuben Wassers, und darüber hinaus einen ausreichenden Zwischenraum für wirksame Belüftung, zu gewährleisten. Gleichzeitig fungiert die strukturierte Oberfläche als vorübergehender Absorber für die abstrahlende Wärme vom Oberkörper und darüber hinaus auch als reversibler, ausgleichender Zwischenspeicher für einen kleinen Teil des zerstäubten Wassers, welches unter Einwirkung der absorbierenden Wärme unmittelbar verdampft und aus diesem Bereich durch Belüftung kontinuierlich entfernt wird. Untersuchungen ergaben, dass die Wärmeabgabe durch Abstrahlung von menschlichem Körper eine wesentliche Rolle spielt. Es wurde z.B. bei ruhendem Menschen bei 20 °C ermittelt, dass die Wärmeabgabe erfolgt durch: Wärmeabstrahlung 46 %, Konvektion 33 %, Schwitzen 19 % und Atmung 2 % (Prof. Dr.-Ing. E. Specht, Uni. Magdeburg, "Der Mensch als wärmetechnisches System", 2005).

Im unteren Bereich des Behältnisses befindet sich das Kühlmodul für den Rücken. Es besteht aus einem oder mehreren Lüftern, einem oder mehreren Ultraschall-Wasserzerstäubern, einem Elektroakkumulator, einem Spannungsregler und/oder einem Spannungs-Impulsregler mit einstellbarer Zeitdauer. Die Lüfter und Ultraschall-Wasserzerstäuber sind nebeneinander angeordnet und mit den übrigen Bauteilen zusammengefasst zu einem Modul, welches aus dem Behältnis herausnehmbar ist.

Man kann alternativ die Ultraschall-Wasserzerstäuber, Lüfter, Spannungsregler und Batterie einzeln direkt an einem nach der Erfindung modifiziertem Hosengürtel an besonders geeigneten Stellen anbringen und als modifizierte und kompakte Version der vorhin beschriebenen autarken Kühlvorrichtung in dieser handlichen Ausführung jederzeit und überall nach Bedarf nutzen zu können. Hierzu wird nach der Erfindung der modifizierte Gürtel mit zwei parallelen Reihen von magnetisierten Nieten, jede Reihe jeweils mit Elektroleiter sowohl miteinander als auch mit der am Gürtel angebrachten Batterie angeschlossen, verwendet. Außer der elektrischen Anschlussmöglichkeit für Ultraschall-Wasserzerstäuber und Lüfter an die Batterie, dient der Gürtel zugleich auch als Träger für diese Kühlbausteine. Zum Zweck der einfachen Befestigung mit gleichzeitigem Elektroanschluss der Lüfter und Wasserzerstäuber an die Batterie an beliebiger Stelle des Gürtels, sind die Nieten in der Reihe jeweils miteinander und mit einem der Batteriepole verbunden. Die Lüfter und Wasserzerstäuber sind jeweils in einem Gehäuse mit Öffnungen für Luft untergebracht. An der dem Gürtel zugewandten Gehäusefläche sind voneinander elektrisch isolierte Neodym-Magnete befestigt, die jeweils zu einem der Nieten in der oberen oder unteren Reihe am Gürtel deckungsgleich positioniert sind. Diese Magneten sind anderseits mit dem Lüfter bzw. Wasserzerstäuber elektrisch verbunden. Die Magneten sorgen nicht nur für eine korrekte und zuverlässige elektrische Verbindung mit der Batterie, sondern dienen gleichzeitig als eine einfache und schnell durchführbare Befestigung am Hosengürtel und zwar an Stellen platziert, an denen der Anwender die Kühlwirkung bei gegebenen Bedingungen als Optimum empfindet. Zum Schutz vor Außenwärme dient die zuvor beschriebene, wärmeisolierende Jacke. Diese sehr vereinfachte, recht kleine und dazu leichte Kühlvorrichtung nach der Erfindung ist dennoch ausreichend leistungsfähig und bei der Nutzung kaum störend. Darum kann sie uneingeschränkt überall genutzt werden.

Zur thermischen Abschirmung des Oberköpers und eines Teils der Kühlvorrichtung dient eine Wärmeschutzhülle in Gestalt eines Umhangs oder einer Jacke aus dem vorhin schon beschriebenen zweiteiligen Aufbau, nämlich einem lichtstreuenden Überzug und einem Licht- bzw. Wärmestrahlung reflektierenden und wärmeisolierenden Schichtverbund darunter. Das Gerüst samt der Installation im Kopfbereich sowie die Wärmeschutzhülle können nach dem Gebrauch wieder zusammengelegt und in dem Behältnis verstaut werden. Die Kühlvorrichtung bildet danach eine handliche, kompakte Einheit und kann wie ein Rucksack transportiert bzw. aufbewahrt werden.

Vorteilhafte Ausgestaltungen der Erfindung sind in den Unteransprüchen angeführt.

So wird vorgeschlagen,
- dass die tragbare Kühlvorrichtung (10) für den Menschen eine derartige Form aufweist, so dass der Lüftungs- und Kühlungsbereich während der Nutzung zwischen dem Behältnis und Körperrücken gebildet wird,
- dass die dem Oberkörper zugewandte Behältniswand derart strukturiert ist, bevorzugt in Form von Noppen, so dass der Lüftungs- und Kühlungsbereich während der Nutzung zwischen dem Behältnis und Körperrücken gebildet wird,
- dass die Kühlvorrichtung für Tiere in Form einer wärmeisolierenden Weste bzw. Decke ausgebildet und deren Innenseiten so strukturiert sind, dass die Kühlung des Tierkörpers darunter möglich wird,
- dass die Kühlung praktisch nur aufgrund der Verdampfung von zerstäubtem Wasser mit Hilfe von Ultraschallwellen und zwar unmittelbar im zu kühlenden Bereich erreicht wird, und die Belüftung mittels Umgebungsluft lediglich zur Austragung des sich bildenden Wasserdampfes dient,
- dass das für die Kühlung verwendete, durch Ultraschall sehr fein zerstäubte Wasser und die für die Austragung des sich bildenden Wasserdampfes durch Belüftung verwendete Außenluft mengenmäßig regelbar sind, um die Kühlleistung der aktuell herrschenden Außentemperatur anzupassen und optimal zu gestalten, insbesondere durch Veränderung der elektrischen Spannung an den Lüftern und/oder den Ultraschall-Wasserzerstäubern mit Hilfe von Spannungsreglern,
- dass die Vorrichtung für die benötigte Elektroenergie zur Belüftung und zum Zerstäuben von Wasser mit einem Akkumulator ausgestattet ist, der insbesondere nach Bedarf aus dem Elektronetz periodisch aufgeladen und während des Betriebes in sonniger Gegend von einem Solarpanel kontinuierlich nachgeladen werden kann,
- dass zur Sicherung einer optimalen Stromausbeute das Solarpanel horizontal und / oder entsprechend dem Sonnenstand über dem Kopf ausrichtbar ist,
- dass zum Schutz vor Außenwärme eine Umhüllung für den Oberkörper und einem Teil der Vorrichtung und zwar in Gestalt z. B. eines Umhangs oder einer Jacke vorgesehen ist,
- dass die Umhüllung einen dreischichtigen Aufbau aus lichtstreuenden, Wärmestrahlung reflektierenden und wärmeisolierenden Verbundschichten aufweist.

### Ausführungsbeispiele

Im Folgenden werden mehrere Ausführungsbeispiele der Erfindung, teilweise anhand von Zeichnungen, näher beschrieben. In allen Zeichnungen haben gleiche Bezugszeichen die gleiche Bedeutung und werden daher gegebenenfalls nur einmal erläutert.

Es zeigen
- Figur 1: eine perspektivische Darstellung der erfindungsgemäßen tragbaren Kühlvorrichtung,
- Figur 2: das Kühlmodul 2 aus Figur 1 und
- Figur 3: eine Seitenansicht der erfindungsgemäßen tragbaren Kühlvorrichtung nach Figur 1.

### Allgemeines

Die Vorrichtungen nach der Erfindung dienen zur kontinuierlichen Körperkühlung von Mensch bzw. Tieren auf ein erträglicheres Niveau während eines Aufenthaltes in besonders warmer Umgebung.

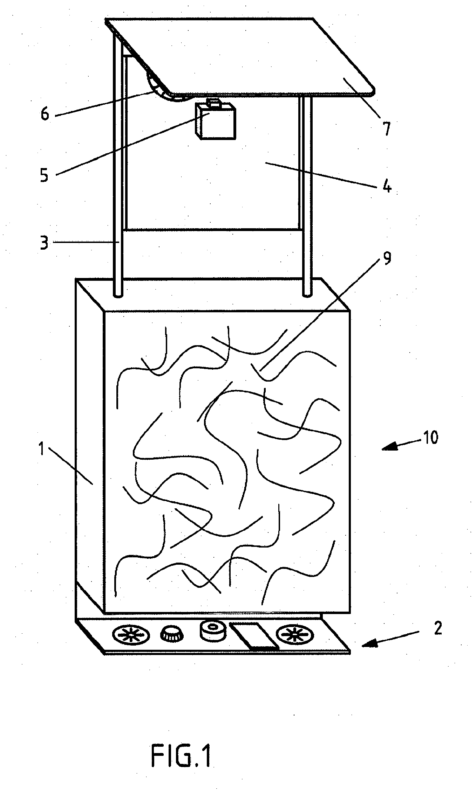

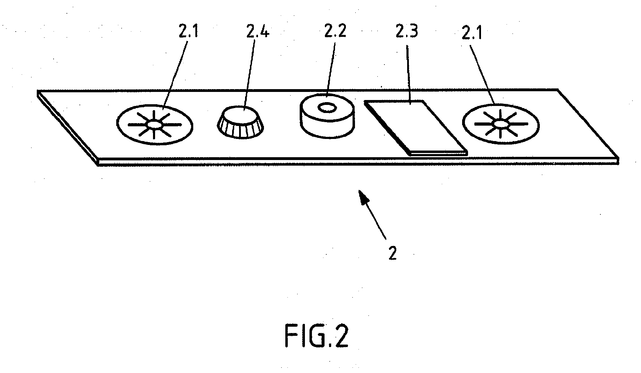

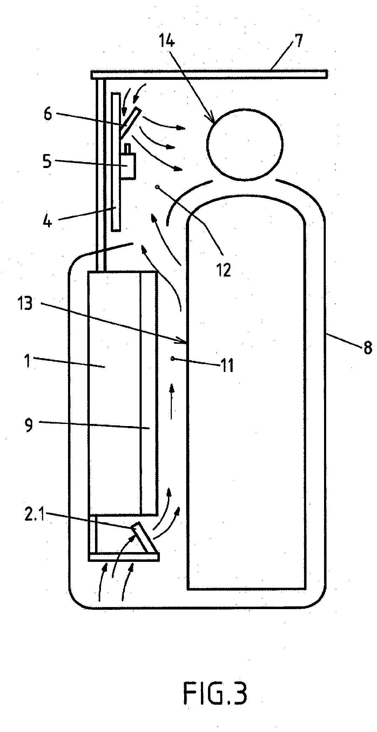

### Beispiel 1 der Kühlvorrichtung für Menschen nach der Erfindung (Figuren 1 bis 3)

Die Vorrichtung für den Menschen nach der Erfindung (Figuren 1 bis 3) besteht aus einem Behältnis 1 mit Tragegurten, einem herausnehmbaren Modul 2 mit zwei Lüftern 2.1, einem Ultraschall-Wasserzerstäuber samt Vorratsbehälter 2.2, einem elektrischen Akkumulator 2.3 und einem Spannungsregler 2.4.

Des Weiteren besteht die Vorrichtung aus einem ausziehbaren Gerüst 3 mit einer darauf montierten Sonnenschutzblende 4 mit einer zusätzlicher Kühlung im Kopfbereich, bestehend aus einem Ultraschall-Wasserzerstäuber samt Vorratsbehälter 5 und einem Lüfter 6, und endet mit einem Solarpanel 7, das über dem Kopf horizontal am oberen Ende des Gerüstes 3 positioniert wird.

Ein sehr wichtiger Bestandteil der Kühlvorrichtung nach der Erfindung ist die Wärmeschutzhülle 8 in Gestalt eines Umhangs oder einer Jacke, die teilweise auch das Behältnis 1 bedeckt.

Die Wärmeschutzhülle nach der Erfindung zeichnet sich durch dreischichtigen Aufbau aus, nämlich aus lichtstreuenden, wärmereflektierenden und wärmeisolierenden Schichten.

Das Kühlprinzip der Vorrichtungen für Mensch und Tiere nach der Erfindung beruht auf besonders effektivem Verdampfen von kontinuierlich fein zerstäubtem Wasser zu Nebel und dadurch Entzug der Wärme, sowohl am Körper als auch im Kopfbereich und anschließender Austragung des entstehenden Wasserdampfes durch die hauptsächlich darauf beschränkte Belüftung mit Außenluft der zu kühlenden Bereiche. Der Zustrom von Wassernebel sowie die Belüftungsintensität können über den Spannungsregler 2.4 nach Bedarf eingestellt werden. Die mit Feuchte angereicherte Luft verlässt kontinuierlich die Kühlzone im oberen Bereich der Umhüllung 8.

### Benutzung der Kühlvorrichtung

Vor der Benutzung der Kühlvorrichtung werden der Elektroakkumulator 2.3 geladen, die Wasserbehälter am Zerstäuber 2.2 gefüllt, die Sonnenschutzblende 4 mit dem Ultraschall-Wasserzerstäuber 5 und dem Lüfter 6 im Kopfbereich und das Solarpanel 7 über dem Kopf positioniert und fixiert.

Nach dem Anbringen am Rücken des Behältnisses 1 mit den darin untergebrachten übrigen Bausteinen der Vorrichtung und dem Überstülpen der Wärmeschutzhülle 8 über den Oberköper sowie das Behältnis 1 und dem Einschalten der Stromversorgung am Akkumulator 2.3 beginnt im Rückenbereich die Kühlung infolge der Verdampfung des kontinuierlich zugeführten, fein zerstäubten Wassers. Der Raum 11 zwischen der dem Rücken zugewandten Behältniswand mit strukturierter Fläche 9 und dem Rücken 13 wird mit Umgebungsluft durchströmt, um die sich bildende Feuchte aufzunehmen und anschließend im Hals- und Rückenbereich der Wärmeschutzhülle 8 zu entweichen.

Die Sonnenschutzblende 4 mit dem darin integrierten Wasserzerstäuber 5 und dem Lüfter 6 sorgt für die Kühlung im Kopfbereich. Die Positionierung des Solarpanels 7 horizontal über dem Kopf gewährleistet dadurch eine optimale Stromausbeute und sorgt für eine kontinuierliche Nachladung des Elektroakkumulators 2.3 und zwar unabhängig von der Bewegungsrichtung des die Kühlvorrichtung tragenden Menschen. Nach Gebrauch wird das Gerüst 3 und das Solarpanel 7 zusammengelegt und zusammen mit der Wärmeschutzhülle 8 in dem dazugehörigen Behältnis 1 untergebracht.

### Beispiel 2 der Kühlvorrichtung nach der Erfindung für einen Hund

Die Weste für Hund kann aus einem wärmeisolierenden Werkstoff bestehen, z. B. aus einem stabilen, elastischen Kunststoff mit mikroskopisch kleinen Gaseinschlüssen und einer Aluminiumbeschichtung an der Außenseite. Die Außenoberfläche der Weste ist mit einem flexiblen Solarpanel versehen. Die Innenseite der Weste ist entsprechend strukturiert, um optimale Kühl- und Strömungsbedingungen zu sichern.

Das Kühlmodul, bestehend aus Lüfter und Wasserzerstäuber, Akkumulator und Spannungsregler ist vorne bzw. hinter der Weste angebracht und an der Unterseite der Weste für Kühlmedien zugänglich. Während des Kühlvorgangs verdampft der im Zerstäuber erzeugte Wassernebel zwischen der Innenseite der Weste und der Körperoberfläche und die überschüssige Wärme wird dadurch kontinuierlich dem zu kühlenden Körperteil entzogen. Der sich bildende Wasserdampf wird durch Belüften des zu kühlenden Bereiches nach Außen ausgetragen.

Die Kühlvorrichtung nach der Erfindung für ein Pferd ist analog wie bei einem Hund, allerdings unter Berücksichtigung der Unterschiede in der Anatomie und der vorgesehenen Tiernutzung. Beim Reitpferd z. B. kann die Kühlvorrichtung aus zwei Segmenten bestehen, wobei die Kühlung hauptsächlich auf den Brustbereich des Pferdes fokussiert wird. Das zweite Segment dient zum Anbringen des Solarpanels und eventuell noch zusätzlicher Kühlmodule.

Nachfolgend werden noch einige wichtige Einzelheiten der Erfindung zusammengefasst.

Die Kühlvorrichtung umfasst Wasserzerstäuber, Lüfter, Behältnis bzw. Weste, einen Elektroakkumulator, Spannungsregler, eine Sonnenschutzblende mit darauf angebrachtem Wasserzerstäuber und Lüfter, ein Solarpanel und eine Wärmeschutzhülle.

Der Wärmeentzug in dem zu kühlenden Bereich erfolgt hauptsächlich durch Zerstäuben von Wasser, bevorzugt unter Einsatz von Ultraschallwellen.

Die zur Belüftung des zu kühlenden Bereiches zwischen dem Körper und dem Behältnis bzw. der Weste eingesetzte Luft dient hauptsächlich zur Austragung des entstehenden Wasserdampfes aus dem Kühlbereich.

Die dem Rücken zugewandte Behältniswand bzw. die Weste der Vorrichtung hat eine oberflächenvergrößernde Struktur, bevorzugt in Form von Noppen.

Das Gerüst dient als Träger für die Sonnenschutzblende mit dem angebrachtem Wasserzerstäuber und Lüfter sowie zur horizontalen Befestigung des Solarpanels über dem Kopf. Das Gerüst lässt sich zusammenlegen und im Behältnis verstauen.

Die wärmeisolierende Hülle 8 besteht bevorzugt aus zwei übereinander angeordneten Teilen. Der obere, ungebundene Teil der Hülle besteht vorzugsweise aus einem lichtstreuenden Gewebe, einer Folie oder einem Verbund beider Materialarten. Der darunter liegende Teil der Hülle besteht vorzugsweise aus einem Gewebe, einer Folie oder einem Verbund beider Materialarten, wobei die nach oben gerichtete Oberfläche zusätzlich eine wärmereflektierende, vorzugsweise eine metallische, und auf der Rückseite eine wärmeisolierende Beschichtung, aufweist.

Die Weste für Tiere besteht vorzugsweise aus einem wärmeisolierenden Kunststoff, bevorzugt mit mikroskopisch kleinen Gaseinschlüssen, und weist eine strukturierte Unterseite und eine metallisierte bzw. andere wärmereflektierende Außenseite auf.

Diese Vorrichtungen dienen zur Abführung von überschüssiger Körperwärme aus besonders intensiv Wärme abgebenden Bereichen, wie Oberkörper und Kopf.

Dabei wird in die zu kühlenden Bereiche kontinuierlich fein zerstäubtes Wasser eingeleitet, verdampft dort rasch und entzieht dabei der unmittelbaren Umgebung des zu kühlenden Körpers intensiv und zugleich effektiv Wärme.

Zusätzlich findet eine kontrollierte Belüftung der zu kühlenden Körperbereiche statt. Der Zweck der Belüftung ist vor allem die Austragung des sich bildenden Wasserdampfes. Im Detail besteht die Ausführung der Vorrichtung für Menschen nach der Erfindung aus einem Behältnis mit angebrachten Tragegurten, Lüftern, Wasserzerstäubern und einem Gerüst zur Befestigung von Kopfkühler sowie einem Solarpanel. Die wirksame Kühlzone wird während der Nutzung zwischen dem Behältnis und dem Körperrücken gebildet. Während des Kühlvorgangs werden der Oberkörper und ein Teil der Vorrichtung vor der Außenwärme durch Anlegen eines wärmeisolierenden Umhanges abgeschirmt. Zusammengelegt bildet die Kühlvorrichtung eine handliche, leichte und jederzeit einsetzbare Einheit.

Die Kühlvorrichtung für Tiere ist entsprechend modifiziert. Ansonsten besteht sie aus gleichen oder ähnlichen Bausteinen und funktioniert auf analoge Weise wie oben beschrieben. Die Vorrichtungen nach der Erfindung sind vorgesehen für Freizeitaktivitäten bzw. Sondereinsätze von Mensch und Tiere, wie Hunde und Pferde, im heißen Gelände.

### Zusammenstellung der Merkmale der tragbaren Kühlvorrichtung für Menschen und Tiere nach der Erfindung

1. Das neuartige Kühlprinzip dieser Vorrichtung beruht auf dem besonders feinen Zerstäuben des kontinuierlich zugeführten Wassers in den Kühlbereich, wo es augenblicklich verdampft und somit der unmittelbaren Umgebung laufend Wärme entzieht. Hierzu besonders geeignet sind Ultraschall-Wasserzerstäuber.
2. Die Entfernung des entstehenden Wasserdampfes erfolgt durch Belüften des Kühlbereiches mit Außenluft und bleibt auf diese Aufgabe beschränkt, um ein Optimum an Kühleffektivität zu gewährleisten.
3. Neben dem Oberkörper wurde auch die Kühlung des Kopfes bei der Kühlvorrichtung mit berücksichtigt, weil die Abgabe der überschüssigen Wärme hauptsächlich in diesen beiden Bereichen stattfindet.
4. Zu diesem Zweck verfügt die Kühlvorrichtung über die Wasserzerstäuber samt Vorratsbehälter und Lüfter, angebracht unten am Körperrücken und zusätzlich im Kopfbereich, wobei deren Leistung und somit auch die Kühlintensität über Spannungsänderung nach Bedarf wählbar sei.
5. Die dem Körperrücken zugewandte Behältnis-, Westen- bzw. Deckenfläche ist besonders strukturiert und dazu geschwärzt, um eine ausreichende Belüftung und optimalen Wärmeaustausch zu ermöglichen und dadurch eine möglichst hohe Kühlwirkung zu gewährleisten.
6. Zum Schutz vor Außenhitze dient eine wärmeisolierende Umhüllung des Oberkörpers samt Kühlvorrichtung, welche über eine wärmeisolierende, Wärme- und Lichtstrahlung reflektierende und streuende Funktion verfügt. Nach Umdrehen der Innenseite nach außen kann, nach Bedarf, die Umhüllung auch als guter Schutz vor Kälte dienen.
7. Die elektrischen Kühlbausteine können zu einer Einheit zusammenfasst werden und lassen sich somit leichter verstauen, hantieren bzw. austauschen.
8. Alternativ können die elektrischen Bausteine über Magneten am modifizierten Hosengürtel befestigt und problemlos elektrisch mit einer Batterie verbunden werden. Diese auf ein Minimum reduzierte Kühlvorrichtung ist dennoch ausreichend leistungsfähig und außerdem nutzbar in praktisch jeder Umgebung.
9. Ein wichtiger Vorteil der Kühlvorrichtung ist die autarke Stromversorgung in sonniger Gegend.

### Orientierende technische Kenndaten

Gleichspannung 5V bzw. 12V
Stromverbrauch bis 10W/h
Wasserverbrauch 50 - 150 ml/h
Benötigte Solarfläche etwa 15dqm (30x50cm)
Maximale Größe der zusammengelegten Kühlvorrichtung: 40x50x5cm
Gewicht: bis 1,2 kg

### Bezugszeichenliste

- 1: Behältnis
- 2: Kühlmodul, Modul
- 2.1: erster Lüfter
- 2.2: erster Wasserzerstäuber
- 2.3: Akkumulator
- 2.4: Spannungsregler
- 3: Gerüst
- 4: Sonnenschutzblende
- 5: zweiter Wasserzerstäuber samt Vorratsbehälter
- 6: zweiter Lüfter
- 7: Solarpanel
- 8: Wärmeschutzhülle, Umhüllung
- 9: strukturierte Fläche
- 10: tragbare Kühlvorrichtung
- 11: Lüftungs- und Kühlungsbereich
- 12: Lüftungs- und Kühlungsbereich
- 13: zu kühlende Oberfläche, Rücken
- 14: zu kühlende Oberfläche, Kopf

## Patentansprüche

1. Tragbare Kühlvorrichtung in Form eines rucksackähnlichen Behältnisses (1) für Menschen oder Weste bzw. Decke für Tiere,
wobei die tragbare Kühlvorrichtung (10)
mindestens einen Wasserzerstäuber samt Vorratsbehälter (2.2),
mindestens einen Lüfter (2.1)
aufweist,
wobei im Gebrauch ein Lüftungs- und Kühlungsbereich (11, 12) zwischen der tragbaren Kühlvorrichtung (10) und der zu kühlenden Oberfläche (13, 14) des Menschen oder Tieres gebildet wird und
wobei die Lüfter (2.1, 6) und die Wasserzerstäuber samt Vorratsbehälter (2.2, 5) zum Belüften und Kühlen dieses Lüftungs- und Kühlungsbereiches (11, 12), insbesondere nebeneinander, angeordnet sind,
wobei der Lüftungs- und Kühlungsbereich zwischen dem Behältnis und dem Körperrücken gebildet wird,
**dadurch gekennzeichnet,**
**dass** ein zweiter Lüfter (6) und ein zweiter Wasserzerstäuber samt Vorratsbehälter (5) im Kopfbereich angebracht sind.

2. Tragbare Kühlvorrichtung nach Anspruch 1,
**dadurch gekennzeichnet,**
**dass** ein Solarpanel (7) über dem Kopf horizontal positioniert ist.

3. Tragbare Kühlvorrichtung nach Anspruch 1,
**dadurch gekennzeichnet,**
**dass** die am Rücken tragbare Kühlvorrichtung (10) eine strukturierte Oberfläche aufweist, so dass der Lüftungs- und Kühlungsbereich zwischen dem Behältnis und Körperrücken gebildet wird.

4. Tragbare Kühlvorrichtung für Tiere nach Anspruch 1, **dadurch gekennzeichnet,**
**dass** die Kühlvorrichtung als Weste bzw. Decke ausgebildet ist und dass die Innenseite der Weste bzw. Decke strukturiert ist, so dass der Lüftungs- und Kühlbereich zwischen der Weste bzw. der Decke und dem Körperrücken gebildet wird.

5. Tragbare Kühlvorrichtung nach Anspruch 1,
**dadurch gekennzeichnet,**
**dass** die für die Belüftung verwendete Umgebungsluft und das zur Kühlung benötigte, zerstäubte Wasser mengenmäßig nach Bedarf regelbar sind.

6. Tragbare Kühlvorrichtung nach Anspruch 1,
**dadurch gekennzeichnet,**
**dass** die Vorrichtung für die benötigte Elektroenergie zur Belüftung und zum Zerstäuben von Wasser und als Zwischenspeicher für die Solarenergie mit einem Akkumulator ausgestattet ist.

7. Tragbare Kühlvorrichtung nach Anspruch 1,
**dadurch gekennzeichnet,**
**dass** als vereinfachte Kühlvorrichtung, anstatt eines rucksackähnlichen Behältnisses, ein Hosengürtel mit zwei Reihen magnetisierter Nieten, jede Reihe polgleich elektrisch verbunden und mit Anschluss für die Batterie versehen, als Träger und Elektroverbindung zugleich für Lüfter und Wasserzerstäuber mit der Batterie dient.

8. Tragbare Kühlvorrichtung nach Anspruch 1,
**dadurch gekennzeichnet,**
**dass** als vereinfachte Kühlvorrichtung die Lüfter und Wasserzerstäuber mit permanenten Magneten paarweise ausgestattet und elektrisch verbunden sind, und zwar polgleich zu dem Batterieanschluss am Gürtel, und so befestigt, dass sie an jedem senkrechten Paar der Gürtelnieten elektrisch korrekte Verbindung mit der Batterie gewährleisten und magnetisch haften bleiben.

9. Tragbare Kühlvorrichtung nach Anspruch 1,
**dadurch gekennzeichnet,**
**dass** sie ein Solarpanel aufweist, das horizontal und/oder entsprechend dem Sonnenstand über dem Kopf ausrichtbar ist.

10. Tragbare Kühlvorrichtung nach Anspruch 1,
**dadurch gekennzeichnet,**
**dass** sie jeweils eine wärmeisolierende, Wärmestrahlung reflektierende und darüber eine lichtstreuende Umhüllung für Menschen sowie für die Kühlvorrichtung aufweist.

11. Tragbare Kühlvorrichtung nach Anspruch 1,
**dadurch gekennzeichnet,**
**dass** die Kühlwirkung hauptsächlich durch Einsatz von Wasserzerstäubern mit Bildung von sehr feinen Wassertropfen von durchschnittlicher Größe unter 20 µm stattfindet.

12. Tragbare Kühlvorrichtung nach Anspruch 11,
**dadurch gekennzeichnet,**
**dass** das Zerstäuben von Wasser bevorzugt unter Einsatz von Ultraschall stattfindet.

## Claims

1. Portable cooling device in the form of a backpack-like container (1) for humans or a vest or blanket for animals,
the portable cooling device (10) having
at least one water atomizer including a reservoir (2.2),
at least one fan (2.1),
during use, a ventilation and cooling region (11, 12) being formed between the portable cooling device (10) and the surface (13, 14) to be cooled of the human or animal, and the fans (2.1, 6) and the water atomizers including the reservoir (2.2, 5) being arranged, in particular next to one another, for ventilating and cooling this ventilation and cooling region (11, 12),
the ventilation and cooling region being formed between the container and the back of the body,
**characterized in that**
a second fan (6) and a second water atomizer including a reservoir (5) are attached in the head region.

2. Portable cooling device according to claim 1,
**characterized in that**
a solar panel (7) is positioned horizontally over the head.

3. Portable cooling device according to claim 1,
**characterized in that**
the cooling device (10) that can be carried on the back has a structured surface such that the ventilation and cooling region is formed between the container and the back of the body.

4. Portable cooling device for animals according to claim 1,
**characterized in that**
the cooling device is designed as a vest or blanket and **in that** the inside of the vest or blanket is structured such that the ventilation and cooling region is formed between the vest or blanket and the back of the body.

5. Portable cooling device according to claim 1,
**characterized in that**
the ambient air used for ventilation and the atomized water required for cooling can be regulated in terms of quantity as required.

6. Portable cooling device according to claim 1,
**characterized in that**
the device is equipped with an accumulator for the electrical energy required for ventilation and for atomizing water and as an intermediate store for solar energy.

7. Portable cooling device according to claim 1,
**characterized in that**
as a simplified cooling device, instead of a backpack-like container, a trouser belt with two rows of magnetized rivets, each row electrically connected with the same polarity and provided with a connection for the battery, is used simultaneously as a carrier and electrical connection for the fans and water atomizers with the battery.

8. Portable cooling device according to claim 1,
**characterized in that**
as a simplified cooling device, the fans and water atomizers are equipped with permanent magnets and electrically connected in pairs, with the same polarity as the battery connector on the belt, and fastened such that they ensure correct electrical connection to the battery on each vertical pair of belt rivets and remain magnetically attached.

9. Portable cooling device according to claim 1,
**characterized in that**
it has a solar panel that can be aligned horizontally and/or according to the position of the sun overhead.

10. Portable cooling device according to claim 1,
**characterized in that**
it has a heat-insulating cover which reflects thermal radiation and, there above scatters light for humans and for the cooling device.

11. Portable cooling device according to claim 1,
**characterized in that**
the cooling effect mainly takes place by the use of water atomizers with the formation of very fine water droplets having an average size of less than 20 µm.

12. Portable cooling device according to claim 11,
**characterized in that**
the atomization of water preferably takes place using ultrasound.

## Revendications

1. Dispositif de refroidissement portable sous la forme d'un
contenant de type sac à dos (1) pour personnes ou de gilet ou de couverture pour animaux,
dans lequel le dispositif de refroidissement (10) portable comporte **au moins un atomiseur d'eau comprenant un réservoir de stockage (2.2),**
au moins un ventilateur (2.1),
dans **lequel, lors d'une utilisation, une zone de ventilation et de refroidissement (11,** 12) est formée entre le dispositif de refroidissement (10) portable et la surface de la **personne ou de l'animal (13, 14) à refroidir, et**
**dans lequel les ventilateurs (2.1, 6) et les atomiseurs d'eau comprenant un réservoir** de stockage (2.2, 5) sont disposés pour ventiler et pour refroidir desdites zones de ventilation et de refroidissement (11, 12), en particulier les uns à côté des autres,
dans lequel la zone de ventilation et de refroidissement est formée entre le contenant et le dos du corps,
**caractérisé en ce**
**qu'un second ventilateur (6) et un second**
**atomiseur d'eau comprenant un réservoir de stockage (5) sont montés dans la zone** de tête.

2. Dispositif de refroidissement portable selon la revendication 1,
**caractérisé en ce**
**qu'un panneau solaire (7) est positionné horizontalement** au-dessus de la tête.

3. Dispositif de refroidissement portable selon la revendication 1,
**caractérisé en ce**
**que** le dispositif de refroidissement (10) portable sur le dos comporte une surface structurée de telle sorte que la zone de ventilation et de refroidissement est formée entre le contenant et le dos du corps.

4. Dispositif de refroidissement portable pour animaux selon la revendication 1,
**caractérisé en ce**
**que le dispositif de refroidissement est conçu sous la forme d'un gilet ou d'une** couverture et que le côté intérieur du gilet ou de la couverture est structuré de telle sorte que la zone de ventilation et de refroidissement est formée entre le gilet ou la couverture et le dos du corps.

5. Dispositif de refroidissement portable selon la revendication 1,
**caractérisé en ce**
**que l'air ambiant utilisé pour la ventilation et l'eau atomisée nécessaire au** refroidissement peuvent être régulés quantitativement selon les besoins.

6. Dispositif de refroidissement portable selon la revendication 1,
**caractérisé en ce**
**que** le dispositif est **équipé d'un accumulateur pour l'énergie électrique nécessaire à la ventilation et à l'atomisation de l'eau et comme réserve intermédiaire pour l'énergie** solaire.

7. Dispositif de refroidissement portable selon la revendication 1,
**caractérisé en ce**
**qu'en tant que dispositif de refroidissement simplifié, au lieu d'un conteneur en** forme de sac à dos, une ceinture de pantalon comportant deux rangées de rivets aimantés, chaque rangée étant connectée électriquement aux mêmes pôles et pourvue **d'une connexion pour** la batterie, sert de support et de connexion électrique **simultanément pour les ventilateurs et les atomiseurs d'eau avec la batterie.**

8. Dispositif de refroidissement portable selon la revendication 1,
**caractérisé en ce**
**qu'en tant que dispositif de ref**roidissement simplifié, les ventilateurs et les **atomiseurs d'eau sont équipés d'aimants permanents par paires et connectés** électriquement aux mêmes pôles que la connexion de batterie sur la ceinture, et fixés de **telle sorte qu'ils assurent une connexion éle**ctrique correcte à la batterie au niveau de chaque paire verticale de rivets de ceinture et restent collés magnétiquement.

9. Dispositif de refroidissement portable selon la revendication 1,
**caractérisé en ce**
**qu'il comporte un panneau solaire orientable** horizontalement et/ou selon la position du soleil au-dessus de la tête.

10. Dispositif de refroidissement portable selon la revendication 1,
**caractérisé en ce**
**qu'**il comporte respectivement une enveloppe calorifuge et réfléchissant le rayonnement thermique et une enveloppe diffusant de la lumière au-dessus de celui-ci pour personnes, ainsi que pour le dispositif de refroidissement.

11. Dispositif de refroidissement portable selon la revendication 1,
**caractérisé en ce**
**que l'effet de refroidissement s'effectue principalement à l'aide des atomiseurs d'eau avec la formation de très fines gouttelettes d'eau d'une taille moyenne inférieure à** 20 µm.

12. Dispositif de refroidissement portable selon la revendication 11,
**caractérisé en ce**
**que l'atomisation de l'eau s'effectue de préférence à l'aide des ultrasons.**
